# EUROPEAN PATENT APPLICATION

(11) **EP 2 201 949 A1**
(43) Date of publication of application: **30.06.2010**
(21) Application number: 08829734.6
(22) Date of filing: 05.09.2008
(51) Int. Cl.: A61K 31/27, A61K 31/573, A61P 19/02

(54) **PHARMACEUTICAL COMPOSITION COMBINING BETAMETHASONE (STEROIDAL ANTI-INFLAMMATORY AGENT) AND METHOCARBAMOL (MUSCLE RELAXANT) AND USE THEREOF IN THE TREATMENT OF MUSCULOSKELETAL DISEASES**

(30) Priority: 07.09.2007 MX 2007011001
(71) Applicant: World Trade Import-Export, WTIE A.G., 6302 Zug (CH)
(72) Inventor: GARCÍA ARMENTA, María Elena, C.P. 45020 Guadalajara Jalisco (MX); SANTOS MURILLO, Josefina, C.P. 44600 Zapopan Jalisco (MX); ÁLVAREZ OCHOA, Victor Guillermo, C.P. 45222 Zapopan Jalisco (MX)
(74) Representative: Carvajal y Urquijo, Isabel
(86) International application number: PCT/MX2008/000120
(87) International publication number: WO 2009/031879

(57) **Abstract**

The invention relates to a pharmaceutical composition comprising the synergic combination of a steroidal anti-inflammatory agent, such as the active principle betamethasone in the form of the pharmaceutically acceptable phosphate and dipropionate salts thereof, and a centrally acting muscle relaxant, such as the active principle methocarbamol, as well as pharmaceutically acceptable excipients, which are formulated in a single dosage unit intended for intramuscular administration. The composition, which is used to control and treat inflammatory musculoskeletal diseases accompanied by muscle contracture, provides improved therapeutic effect, faster action in less time and a reduction in the risk of side effects, such as drowsiness and inability to perform daily tasks.

## Description

### FIELD OF INVENTION

The present invention has application in pharmaceutical industry and describes a pharmaceutical composition comprising the synergistic combination of a steroidal anti-inflammatory agent, such as active principle: Betamethasone, in form of its pharmaceutically acceptable phosphate and dipropionate salts, and a centrally acting muscle relaxant, such as active principle: Methocarbamol, in addition to pharmaceutically acceptable excipients; which are formulated in a single dosage unit to be intramuscularly administered; indicated for control and treatment of musculoskeletal inflammatory diseases associated with muscle contracture.

The combination of above mentioned active principles produces a larger synergistic effect when administered together with a single dosage unit unlike when they are independently administered, causing benefits such as: lower concentrations of active principles comprised in the formula, lower administered doses, faster pharmacological action and enhancement of therapeutic effect in shorter time and lower risks of showing side effects such as: drowsiness e ambulatory impairment to perform daily activities.

### BACKGROUND OF INVENTION

Arthrosis, also called Degenerative Joint Disease, more than a disease, is a very frequent syndrome which is manifested as the final expression of alterations produced in joints by a varied set of ethiopathogenic processes (excessive or repeated mechanical overload, genetic factors, inflammatory or metabolic processes), being the most common cause of articular disease.

The main feature of degenerative joint process is a hyaline cartilage progressive impairment in body movable joints (diarthroid), both from axial skeleton (spine cord) and peripheral (especially hands, knees and hip); also' accompanied by an affectation of periarticular tissues (deformation, bone hyperthropy, muscle athrophy), being very particular the absence of general or systemic manifestations.

An injury occurs in cartilage and secondarily affects neighbor bone structures (subchondral bone sclerosis, bone reactive formation in joint margins: osteophytes, pseudocysts or geodes and in bone epiphyses close to joint surface), joint capsule and synovial membrane. In parallel with cartilage degeneration an inflammatory reaction of synovial is produced, with a slight cellular infiltration and vascular proliferation contributing to disease progression.

Although progressively advancing degenerative changes are produced with aging, only in a small percentage of cases clinical manifestations appear due to arthrosis. There are risk factors for unchaining a clinical arthrosis, such as: systemic (sex, age, race and genetic determinants) and local (obesity, prior articular injury, repeated microtraumatisms, metabolic disorders and static alterations).

Independently from causal factor, there is an unbalance in arthrosis between degradation physiological processes and those of repair of extracellular cartilage matrix, being catabolic chondrocyte processes predominating over anabolic processes thereof.

Proteolytic enzymes, mainly chondrocyte metalloproteases and synovial cells, are the main activators in destructive process in addition to some cytokines, essentially IL-I and FNT alpha, which also contribute to catabolic process.

Arthritis is a joint affection due to synovial membrane inflammation (synovitis), which is produced as a response to several mechanisms (infection, crystal deposition and immune phenomena). In some diseases, called destructive arthropathies, the inflammatory reaction exceeds synovial limits and secondarily produces cartilage and joint bone end injuries, but in many cases they do not cause osteocartilaginous destruction and they do not show radiologic manifestations.

In microcrystalline arthritis, the crystals having more clinical importance are monosodium urate (gout) and calcium pyrophosphate (chondrocalcinosis or pseudogout).

Spondyloarthropathies are a group of diseases, which paradigm is ankylopoietic spondylitis of unknown etiology with effect over axial joints (sacroiliac and spine cord) and/or peripheral (oligoarticular and assymetric pattern) associated with enthesitis (inflammatory reaction to muscle insertions manifested as a response to suffered traction overload wherein produced damage affects to tendon, bone and cartilage structures). This disease is characterized by an association with a common genetic marker, HLAB27, and the concomitant presence of mucocutaneous, ocular, intestinal and genitourinary affectation.

Soft tissue rheumatisms are painful syndromes which are caused in non-joint locomotive apparatus structures, which diagnosis is mainly clinical.

Located soft tissue rheumatisms such as: entesopathies (enthesis = insertion zone in muscles, ligaments, joint capsules and bone tendons), more frequently manifested are: epicondylitis, epitrocleitis and fascitis plantar. They usually are a consequence of traumatic processes or mechanical overload, but they are also frequent in spondyloarthropathies and in microcrystalline arthritis.

Regional soft tissue rheumatisms, such as myofascial syndrome is characterized by showing located pain and rigidity in a certain portion from locomotive apparatus, having as main feature the presence of "trigger" points or deep location pain triggers which may be identified as muscle or fascia indurations which when pressed unchain pain symptomatology.

Generalized soft tissue rheumatisms such as fibromyalgia and ligament hyperlaxitude or benign joint hypermovility syndrome, are transmitted through AD heritage and predispose to acquired deformities such as: flat feet or scoliosis.

Tendinitis is a painful inflammatory process located in tendons or in synovial sheath, respectively. Tendons are thick fibrous cords through which muscles are inserted into bones; which are surrounded by a kind of "coating or sheath" called sheath, coated internally by synovial tissue. Between tendon and sheath there is synovial liquid which eases tendon sliding within it. Tendon function is to transmit the force generated by a muscle contracture for bone movement. Under situations of mechanical irritation or overload, synovial liquid production may be increased, leading to tenosynovitis, which appears along inflammatory diseases, frequently caused by traumatisms.

In bursitis, the most frequent injury cause is traumatic, although it may be also affected by inflammatory, tumoral or infectious diseases. Bursitis is inflammation or irritation of a "burse", which are small bags located between the bone and other movable structures such as muscles, skin or tendons and which ease a soft sliding of those structures.

Both tendons and bags are proximately situated to joints, therefore an inflammation of those structures is perceived by patients as joint pain and may be mistakenly interpreted as arthritis.

Entrapment neuropathies are produced by a mechanical irritation of certain peripheral nerve which is locally injured in an anatomical vulnerable zone.

Located fibrosis, also known as Dupuytren disease, is characterized by a thickening of palmar fascia and retraction associated with fourth and fifth fingers. Said disease is of unknown cause.

Reflex sympathetic dystrophy or Sudeck syndrome is characterized by the presence of pain and tumefaction in a distal part from a limb associated with vasomotor instability signs and symptoms (sweat, coldness).

Pain is the most frequent form by locomotive apparatus diseases to be present and it is the main symptom. In all cases there is an emotional component which may be very variable, since while some individuals are constantly looking for a relief for daily life common pain, others suffer from pain caused by severe diseases or injuries which demand urgent attention.

A pain which is generated in surface structures such as skin is located over the affected area, but when deep structures are affected such as joints, tendons, ligaments and periostum, pain may be experienced over affected structure or over a distant site.

In order to locate the anatomical injured structure which causes pain, a distinction between the following types of pain is useful:

Surface pain: it is produced by stimuli which act over skin, mucose or nervous fibers innervating them. It is acute, sharp, burning and when persistent it causes a burn sensation.

We may experience an example of this kind of pain by pulling the hairs from hand back. Whichever the causal stimulus, it is always of the same quality.

It is precisely located and accompanied with some associated phenomena such as: cutaneous pain upon pressure, hyperalgesia and parestesia.

Deep pain: it is caused in deep structures such as muscles, bones, joints and internal organs. It is a dull pain such as that felt when a muscular abdomen is pinched, usually vague, wrongly located and frequently perceived at certain distance from affected structure.

Referred pain: It is a deep pain perceived in a structure other than that suffering an injury. It has a segmental distribution belonging to the same neurological segment of injured structure. A distribution over skin of a neurological segment is called dermatome. It is accompanied with painful points when pressing over its area and in order to be distinguished from injury painful point causing pain, the two principles from Lewis and Kelgren are useful (pressure over a referred pain point only causes local pain; however, pressure over an injured point causes local pain + pain in referred pain area; and infiltration with local anesthesia in a referred pain point only produces local anesthesia and infiltration of injured point suppresses local pain and all the area of referred pain).

The most common musculoskeletal referred pains are manifested in: cervical region with origin in the first two cervical vertebrae, C1 and C2, wherein pain is located on head, especially in the nape and in vertex; in C3 and C4 vertebrae, wherein pain is located in injured zone, in upper beams from trapezius muscle and in shoulder; and in C5, C6 and C7 vertebrae, wherein pain is located in large zones of referred pain, such as shoulder, arm and forearm, in scapular and pectoral regions.

Upper limb: pain originated in deep shoulder structures is referred to arm, seldom to forearm and in some cases (rotator cuff calcifying tendinitis) may reach hand fingers. Pain proceeding from elbow is locally felt and occasionally refers to forearm. Forearm and hand aches are usually well located, particularly when injury is more peripheral.

Trunk: pain by trunk injuries, in chest wall and in abdominal wall is usually correctly located. Deep parts from dorsal rachis may lead to referred aches of two kinds: a) surface pain in descendent belt, chest or chest-abdominal which follows the dermatome corresponding area and b) deep pain in horizontal plane which is directly projected over chest outer part. Diaphragm injuries are expressed by referred pain, the central part which is innervated by the phrenic muscle proceeding from C3 and C4 segments, causing pain referred to neck and sometimes to shoulders; the peripheral part innervated by fibers proceeding from the six last intercostal nerves which derive from D6 to D12, causing referred pains to chest lower part and abdominal walls.

Lumbar region - Aches resulting from this zone may be well located or referred to following lower limbs being very frequent that last lumbar vertebrae cause a referred pain in a plane almost horizontal; a typical example is sciatic nerve which is irradiated from gluteus, inner side of thighs and leg.

Pelvis: Two deep structures may be a cause of referred pain in this zones, these being: a) sacroiliac joint which in addition to local pain causes pain referred to inner side of thighs (sciatic) and occasionally at groin; and b) hip, which in addition to groin pain, it may cause that pain is referred to thigh, knee and trochanter. Lower limb: pain generated in thigh, knee, leg, heel and foot tend to be well located. Aches located in deep proximal parts may sometimes generate distal referred pain.

Radicular pain: Those pains due to irradiation or compression of a nerve root causing surface pain irradiated to its dermatome (exact location in radicular band which when affecting to sensitive fibers, it is accompanied with sensitive alterations such as: hypoalgesia or hyperalgesia, paresthesia, tingling, sharp pain, numbness, cold and hot sensation), which in some cases do not correspond exactly with dermatome, causing that deep pain is present in zones other than dermatome, for instance, in shoulder blade or pectorals in cervico-brachial origin injuries which cause a confusion with cardiac or pulmonary origin pain.

Muscle contracture is a body defense mechanism which paralyzes a specific zone thereof, which is produced by contracting involuntarily and persistently certain muscles or muscle groups, causing pain and burning without being severe. Causes are several being those more common: extreme temperatures, action of certain drugs or local accumulation of lactic acid, caused by unusual stresses. Many cases of muscle contracture are presented in cervical, dorsal and lumbar cord due to incorrect positions or sudden movements. It is commonly manifested by a heaviness sensation in affected zone, in addition to a difficulty in moving close joints.

There are two types of muscle contractures: a) those which appear when any exercise is being carried out, and b) those which are present after stress.

Those from the first type are due to an accumulation of metabolic products within muscle tissue. When a muscle starts to work it demands energy which is provided by blood flow which transports nutrients collected from food; these substances react with oxygen within the muscle and remove the necessary energy for muscle fiber contracture. Muscles are oxygenated and fed through blood flow and resulting toxic substances are removed. When an intense and unexpected movement is performed, blood vessels on one side are not sufficiently developed or dilated as to nourish the muscle under work and on the other hand, they are insufficient to clean muscular fibers from produced toxic wastes; when these toxic elements are released they cause at the same time, pain and contractures in the affected muscle.

Another contracture form also frequent is that from second type, which appear after physical exercise, generally caused by any muscular fiber being distressed or subject to an excessive workout. An excessive distress or a direct traumatism may cause injuries over muscle tissues or on joints. Injured tissue is repaired within a few days or in more severe cases, within two or three weeks, disappearing the pain and difficulty to move.

Nowadays, a high musculoskeletal inflammatory disease prevalence has been detected which have caused an increase in hospital (presence of permanent pain and physical impairment or limitation) and economical (drug consumption and labor sick leave) expenditure in the country.

Most of drugs which are found in marketplace for treatment of musculoskeletal inflammatory diseases associated with muscle contracture comprise active principles which are independently formulated, which fulfill with a so specific therapeutic activity, that in most cases is limited as to the number of symptoms which are manifested in a musculoskeletal inflammatory process, in addition to pain and muscle contracture as a consequence thereof.

### SUMMARY OF INVENTION

Pharmaceutical industry is currently conducting a significant effort to achieve pharmaceutical product development which favorably has influence in musculoskeletal inflammatory disease recovery which is accompanied by muscle contracture.

In order to offer a pharmaceutical alternative to achieve a removal of musculoskeletal inflammatory process, the pain as a consequence thereof and muscle contracture, development of present invention was carried out, whereby a pharmaceutical composition comprising a combination of a steroidal anti-inflammatory agent and a centrally acting muscle relaxant is described, those which act synergistically and which are formulated in a single dosage unit to be intramuscularly administered, which provides significant benefits such as: lower concentrations of active principles comprised in the formula, lower administered doses, enhancement of therapeutic effect and faster pharmacological action in a shorter time, as well as a risk reduction of manifesting secondary effects such as drowsiness and ambulatory impairment to perform daily activities.

### DETAILED DESCRIPTION OF INVENTION

Steroidal anti-inflammatory agents, also known as corticosteroids, are hormones produced or synthesized by adrenal cortex (natural corticosteroids) or from semi-synthetic origin (being structural analogs of natural corticosteroids). They are indicated in every rheumatic inflammatory disease; however, they are also clinically used such as: substitutive therapy, immunosuppressants and anti-inflammatories. The main indications are the existence of a severe organic effect disease and previously uncontrolled arthritis with non-steroidal anti-inflammatories (NSAIDs) and remission inducing drugs. Natural corticosteroids are classified in two kinds:
a) Mineralocorticoids: These are produced in glomerular zone. They are responsible of hydrosaline equilibrium regulation. Aldosterone and corticosterone belong to this group of corticosteroids.
b) Glucocorticoids: These are produced in fasciculated zone. They are responsible of controlling carbohydrate, fat and protein metabolism. Cortisol (hydrocortisone) belongs to this group of corticosteroids.
   Natural corticosteroid biosynthesis is present from synthesized cholesterol using acetate or from cholesterol obtained from systemic circulation. Mechanisms which regulate steroid biosynthesis and release are the corticotrophin release factor (ACTH) in case of glucocorticoids, which action is manifested at adrenal cortex level, wherein calcium (Ca++) and cyclic nucleotides have an important role in said mechanisms; and in case of mineralocorticoids, its regulation mechanism is through renin-angiotensin system.

Aldosterone is the main mineralocorticoid hormone in human body. It causes sodium (Na+) and H2O retention in kidney distal tubule and an increase in potassium excretion (K+). Main glucocorticoid hormone in humans is cortisol.

Semi-synthetic corticosteroid are obtained through a modification of chemical structure of natural corticosteroids (cortisone or hydrocortisone), through the introduction of double linkages (called delta corticosteroids) in groups OH, CH3, Fl; thus increasing potency, decreasing mineralocorticoid and improving glucocorticoid activities. Under this group we may find to: prednisone, prednisolone, methylprednisolone, dexametaxone, and others.

Steroidal anti-inflammatory agents are intracellularly located. Steroids penetrate in cells by passive diffusion and they are fixed in an ectoplasmatic specific receptor. In a second phase, the activated receptor-steroid complex, suffers from an activated translocation process and is fixed to core DNA leading to RNA formation and the corresponding protein synthesis which lastly, mediates steroid physiological and pharmacological effects.

Steroidal anti-inflammatories show good oral absorption, excluding aldosterone and DOCA (desoxycorticosterone). Soluble salts (phosphates, succinates) are rapidly absorbed and insoluble salts (acetates) are slowly absorbed by intramuscular route. Soluble salts are intravenously administered being of fast action in emergency cases. Physiological steroids (cortisol) circulate joined to a transporting protein in 95% (transcortin). Those synthetic are linked to proteins in lower degree. They are metabolized in liver and removed through kidney. Synthetic steroids are slowly metabolized.

Steroidal anti-inflammatories are classified according to its action type, this being: short action such as cortisol; intermediate action such as prednisolone and metilprednisolone and long action such as betamethasone and dexametasone.

Among pharmacological effects from steroidal anti-inflammatories are: promoting glucogen accumulation on liver and stimulating gluconeogenesis; decreasing glucose peripheral uptake (skeletal muscle, lymphocytes, fatty and conjunctive tissue); fat redistribution specially those deposited over face (full moon face) and neck (buffalo hump), showing a significant lipolytic effect; increasing Na+, H2O and anion reuptake at distal tube level, in addition to an increase in K+ removal; decreasing circulating lymphocyte presence (including T cells), eosinophils, basophils, monocytes and macrophages and increasing the number of circulating neutrophils, erythrocytes, platelets and hemoglobin; reducing fracture consolidation; they may cause euphoria and sometimes depression.

Corticosteroid anti-inflammatory effect is related with a decreased production of prostaglandins and leukotrienes, representing the key basis of clinical use, since it inhibits vasodilatation, increases vascular permeability, exudation and cellular proliferation which appear in inflammatory processes. The anti-inflammatory effect is unspecific and independent from the unchaining agent, whether physical, chemical or infectious.

The best use of a steroidal anti-inflammatory is by using low doses thereof to achieve symptom and articular inflammatory sign control, such as pain and muscle rigidity, when pure analgesics and NSAIDs fail at the beginning.

Corticosteroids are commonly available in a number of pharmaceutical forms, such as: oral, topical, ophthalmic solutions and ointments, oral inhalators, nasal formulations, parenteral and rectal.

Intravenous and intramuscular absorption depends on used agent solubility. Additional administration routes for parenteral corticosteroids include: intra-articular, intrabursal, intrasynovial, intralesional and subcutaneous. Systemic absorption has been reported in all above mentioned routes.

Prolonged administration of glucocorticoid physiological doses in replacement therapy does not cause adverse reactions; however, the severity of adverse effects associated with a prolonged treatment increases doses and treatment duration. Short-term treatments usually do not produce adverse reactions, but long-term treatments may cause athrophy in suprarrenal glands and adrenocortical generalized depletion.

Glucocorticoids are responsible of proteinaceous metabolism, therefore a prolonged treatment may cause several musculoskeletal manifestations (myopathy, myalgia), bone matrix athrophy and healing delay. They also interact with calcium at several levels, particularly decreasing the bone matrix protein synthesis by osteoblasts and reducing calcium absorption intestinally and over nephrons, however, they do not modify vitamin D metabolism. Betamethasone intra-articular injections may cause arthropathies similar to those from Charcot, and athrophies may also be found in injection points including tendon rupture.

Betamethasone and its pharmaceutically acceptable salts, such as Betamethasone phosphate and Betamethasone dipropionate, are prolonged action synthetic glucocorticoids which are used as anti-inflammatories and immunosuppressant agents. Betamethasone pharmacological dose administration achieves an inflammation reduction by inhibiting acidic hydrolase release which are found in leukocytes thus preventing the accumulation of macrophages in infected places, interfering with leukocyte adhesion in capillary walls and reducing the permeability of capillary membrane, which causes an edema reduction. Moreover, Betamethasone reduces the concentration of complement components, inhibits histamine and kinin release, and interferes with fiber tissue formation.

Betamethasone anti-inflammatory activity is due to its effect over lipocortins, phospholipase A2 inhibiting proteins, which control the synthesis of potent inflammation mediators such as leukotrienes and prostaglandins, acting by inhibiting the prodrug, arachidonic acid synthesis.

Betamethasone immunosuppressant activity is due to an alteration over lymphatic system performance, which reduces immunoglobulin and complement concentrations, inhibiting immunocomplex transportation through capillary membranes, in addition to reduce lymphocyte count and to interfere in antigen-antibody reactions.

Betamethasone is orally administered, whereas Betamethasone phosphate may be administered by intravenous, intramuscular, intrasynovial, intraarticular or intralesional route.

After oral administration, Betamethasone is rapidly absorbed. Maximum plasma levels are reached when elapsing 1 to 2 hours after an oral dose is administered. Action beginning and duration of Betamethasone suspensions depend on the administration route (intramuscularly, intraarticularly, etc) and on local blood irrigation where infiltration has been performed; for instance, after intraarticular administration, the drug slowly pass generally into circulation. Betamethasone absorption through skin depends on integrity thereof on application place and application area, since it is increased when skin is injured, inflammated or when administered by an occlusive bandage and it is also higher in zones where stratum corneum is thinner such as eyelids, genitals or face.

Systemically administered betamethasone is rapidly distributed in kidneys, intestines, skin, liver and muscles. The drug is weakly joined to plasma proteins, being only active a part which is not joined to said proteins. Generally, corticoids and therefore Betamethasone, traverse placental barrier and are excreted through mother milk. Topical betamethasone is locally metabolized in skin while systemic betamethasone is metabolized in liver, producing inactive metabolites. These are excreted through urine together with a small drug amount without metabolizing. Betamethasone half-life is about 35 to 54 hours. Centrally acting muscle relaxants act over internuncial neurons suppressing polysynaptic paths, exerting their action over upper autonomous nerve centers. They are also used as tranquilizing agents.

Muscle contracture (CM) is manifested when a sudden, violent and involuntary increase in muscle stress is present, as a result of traumatisms, inflammatory type alterations and other type of lesions in skeletal muscle. It is a more or less lasting state which causes pain or affects both under rest and movement conditions, limiting the joint performance related with affected muscle or muscular group.

Muscle contracture involves a nociceptive afferent conduction from damaged tissue, stimulated by alpha-motor neurons, to muscle, leading to a painful tonic contracture of affected muscle, creating a vicious circle, since pain caused by spasm causes a larger nociceptive stimulus which upon exciting alpha-motor neurons, causes more spasm and pain.

Under muscle contracture physiopathology, descending tract dysfunction' (corticospinal, vestibulospinal, reticulospinal) which exerts control over alpha-motor neurons is also included. Peripheral reflex arcs, although hyperactive because of abnormal control received from upper nerve centers, apparently do not intervene in pathological process; however, some sites where these arcs have influence, including afferent nerve fibers proceeding from skin and muscle spindles, spine cord interneurons, efferent nerve fibers and intrafusal and extrafusal muscle fibers, use to be blanks for pharmacological intervention which shall control an increase in muscle tone.

Physical-sport activity causes a number of situations leading to pain in locomotive apparatus. Besides the circumstances due to articular and bone injuries, the presence of local pains in muscle masses occupies a remarkable place within the main causes of medical visit.

Myalgia is quite inaccurate and therefore referred to as cramps, contractures, spasms and a number of terms which occasionally do not define specific situations. Sometimes the injury origin allows better specificity and then is called a fibrillar breakage or contusion, by acknowledging a concrete traumatism as a cause of muscular pain.

Physiological mechanisms of contracture and innervations proper of muscle-tendon explain pain physiopathology. They are of special importance: defective relaxation, modification of tone control by gamma loop, participation of alpha-motor neurons and nociceptive innervation, in addition to a significant role performed by microcirculation.

Muscular processes having pain show an intimate inflammation substrate or interstitial local edema. The fact that an increase in tissue or contracture is frequently apparent makes generally that medical treatment in these situations demand the use of muscular relaxing agents together with analgesics and anti-inflammatories.

Sport practice involves a number of muscular pain situations due to traumatic, microtraumatic or overload aggressions. Several causes comprise from acute traumatism which injures because of its intensity, to the small stress which injures because the muscle is tired or overloaded. In all cases, pain is present spontaneously upon palpation and with muscle function.

Relaxing drugs of skeletal musculature are used for treatment of pathological processes associated with muscle contractures and they have the main purpose of normalizing the muscle excitability without deeply affecting the motor function. Muscular relaxing agents shall not prevent contracture but instead normalizing excitability and muscular tone to decrease pain and to improve motor function.

Methocarbamol is an analog carbamate derived from mephenesin producing polysynaptic reflex inhibition, which performs as central action muscular relaxing. The beginning of Methocarbamol pharmacological action occurs within 30 minutes after being orally administered, achieving its maximum effect within 3 to 4 hours after delivery, with a total therapeutic action duration of about 24 hours. Its half life is about 14 hours.

Methocarbamol is almost fully absorbed after being orally administered; it is metabolized by dealkylation and hydroxylation and its removal is performed mainly through urine as glucuronide and sulfate conjugates of its metabolites, only a small amount thereof is excreted through feces.

Methocarbamol action mechanism has not been yet well determined; however, results from research performed in animals have demonstrated that it acts by reducing the reflex polysynaptic activity in spine cord, and it is through this mechanism that achieves a reduction in alpha-motor neuron excitability, thus providing a result in skeletal muscle; moreover showing a central nervous system depressive effect, leading to a sedation state which is mostly responsible of the muscle relaxing effect.

It is used as an adjoined therapy in treatment of symptoms manifested by suffering painful muscular spasms.

Usual used dose for oral route methocarbamol is up to 1.5 g four times a day, then reducing to a maintenance dose of 4 g per day during 2 to 3 days. A dose of 750 mg, three times a day has been ensured to be sufficient to have an efficient therapeutic effect.

Methocarbamol may be also intramuscularly administered with a dose up to 500 mg every 8 hours, a 300 mg per minute dose intravenously in a slow injection or by infusion in sodium chloride or glucose. Parenteral dose shall not exceed 3 g daily during 3 days.

There are currently a number of pharmaceutical products in marketplace directed for treatment of musculoskeletal inflammatory diseases, wherein a number of active principles are found which are independently formulated; however in this kind of disorders it is quite important to prevent complications which are present together with the disease, such as: intense pain and muscle contracture, as well as reducing the risk of manifesting side effects such as drowsiness and impairment to perform daily tasks; therefore, the activity of several drugs acting by different mechanisms is indispensable to shorten both painful and inflammatory process such as ambulatory impairment caused by said diseases.

The pharmaceutical composition subject of the present invention comprises a synergistic combination of a steroidal anti-inflammatory agent, such as active principle: Betamethasone, in form of its phosphate and dipropionate pharmaceutically acceptable salts, and a centrally acting muscle relaxant, such as active principle: Methocarbamol, in addition to pharmaceutically acceptable excipients, those which are formulated in a single dosage unit to be intramuscularly administered, which is indicated for control and treatment of musculoskeletal inflammatory diseases associated with muscle contracture; and having been developed taking into account that said active principles have a great potency and efficacy to prevent or to decrease the symptomatology which is associated with those diseases such as: intense pain and inflammatory process, in addition to arrest muscle contracture progression, reducing the time where therapeutic effect is manifested and to prevent or decrease the appearance of secondary effects such as: drowsiness and ambulatory impairment to perform daily activities.

The steroidal anti-inflammatory agent used in the pharmaceutical composition subject of present invention such as active principle: Betamethasone, is present in the formulation in the form of its phosphate and dipropionate pharmaceutically acceptable salts which are present in a concentration range from 2.0 mg to 4.0 mg y from 5.0 mg to 10.0 mg, respectively, per dose unit.

The centrally acting muscle relaxant used in the pharmaceutical composition subject of the present invention, such as active principle: Methocarbamol, is present in the formulation in a concentration range from 100.0 mg to 750.0 mg per dose unit.

In order to assess the efficacy and tolerance of the pharmaceutical composition subject of the present invention, as well as a synergistic effect of active principles Betamethasone phosphate, Betamethasone dipropionate and Methocarbamol combined in a single dosage unit, a comparative clinical study was carried out wherein above mentioned active principles were separately administered, as well as a combination thereof.

### ANTI-INFLAMMATORY AND ANALGESIC EFFECTS OF BETAMETHASONE + METHOCARBAMOL IN A PIFIR EXPERIMENTAL MODEL.

PIFIR experimental model is a preclinical model wherein gout arthritis is possible to be determined in a rodent and the anti-inflammatory and analgesic efficacy of several drugs in animals with this alteration is compared. This experimental model has some important advantages regarding other preclinical methodologies: a) an alteration which clinically affects to humans is established in the rodent: gout type arthritis; b) temporary course of anti-inflammatory and analgesic effect may be determined along 4 continuous hours in the same experimental subject, without producing rodent conditioning factor or learning, being this a significant disadvantage in other experimental models; c) less test drug is consumed compared with other experimental models; d) anti-inflammatory and analgesic effect assessment is performed by computer and not depending on sometimes subjective observations.

It has been demonstrated for a more accurate detection of the potential or actual utility of drug associations that a Synergistic Action Surface analysis is very important to be performed, since an assessment of these drug associations in different association ratios is capable of producing a range of effects from antagonism to enhancement, including a single effect sum.

In the light of the above, object of this work was to compare the anti-inflammatory and analgesic effects produced both individually and in association with, of a single steroid anti-inflammatory dose of Betamethasone, and a centrally acting muscle relaxant, Methocarbamol, in a PIFIR experimental model.

### MATERIALS AND METHODS.

Experiments were performed by using female Wistar rats having a weight of about 180 to 200 g. Animals were kept in a room with alternate light/darkness cycles. Food was withdrawn from rats twelve hours before experimentation, with water ad-libitum. All experiments were carried out during light time, the animals being used only once.

Betamethasone and Methocarbamol were firstly dissolved in 0.75 mL. of alcohol and then carried to a final volume of 3.0 ml with 0.5% carboxymethylcellulose.

Animals were anesthesized in a glass desiccator saturated with ester vapors. Gout arthritis was induced by applying a 0.05 ml intra-articular uric acid injection suspended in mineral oil in the right anterior limb, exactly on the femoral-tibio-rotular joint. A 1 ml glass syringe was used for intra-articular injection with a No. 22 4 mm long needle. An electrode was fixed immediately later in every anterior leg between the plantar calluses. Rats were left to recover from anesthesia and they were enclosed in a 30 cm diameter stainless steel rotary cylinder. Cylinder was rotated at 4 rpm, forcing to the rats to walk during 2 min every half hour, during 5 hours after total dysfunction.

Contact time was the measured variable at every anterior leg from the rats in the cylinder. When electrode is in contact with the cylinder a circuit is closed and the contact time ratio between injured to uninjured legs was registered in a computer.

Anti-inflammatory and analgesic effects caused by Betamethasone and Methocarbamol were individually studied, gout arthritis being already established in rats. Assessed doses for each of the compounds were as follows: Betamethasone 177.8 mg/kg and Methocarbamol 177.8 mg/kg

Anti-inflammatory and analgesic effects caused by these compounds in the same doses (above mentioned) where simultaneously determined, but in combined administration, Betamethasone + Methocarbamol.

Temporary courses for every treatment were determined during 5 continuous hours, using a "n" of 6 rats per treatment.

### RESULTS

Uric acid induced a complete right front limb dysfunction of about 2.5 hours after delivery, corresponding to a zero IF% value. Rats which received vehicle (mineral oil or carboxymethylcellulose) did not show any IF% recovery during the 5 hour observation period (data are not shown here). Betamethasone used dose did not affect the walking rat capability during the observation period. Methocarbamol used dose did not impair assessment performance; however, a slight relaxation effect was caused in animals.

CT developed by Betamethasone in 177.8 mg/kg doses is noticed to generate limited anti-inflammatory and analgesic effects, with a typical CT profile for steroid type anti-inflammatory compounds, that is, a quite delayed action start, but slowly growing along time. Maximum effect was present at 4.5 hours after delivery (24.0 ± 11.9%).

CT developed by Methocarbamol in 177.8 mg/kg doses is observed to generate a larger result variability. Slight relaxation effects were manifested in rats showing that rats were slower when walking and in this way, they made contact with both legs when walking.

CT developed by a combination of Betamethasone + Methocarbamol in above mentioned doses is noticed to cause a significant increase in anti-inflammatory and analgesic effects. After de 1st hour of administration, the anti-inflammatory and analgesic effect grows significantly, still maintained after 5 hours of delivery with a large anti-inflammatory and analgesic effect (73.2 ± 12.2%).

Maximum effect was reached 3 hours after delivery: 79.0 ± 6.1%.

It is very clear that the simultaneous administration of both active principles produces a faster generation of anti-inflammatory and analgesic effects and importantly improves their efficacy.

Global anti-inflammatory and analgesic activity through 5 hours of assessment expressed in CT AUC: Betamethasone produced a 31.6 ± 10.8 ua effect and Methocarbamol 48.8 ± 21.0 ua; while the combination of Betamethasone + Methocarbamol produced a 223.1 ± 13.2 ua effect.

PIFIR model was used since it allows anti-inflammatory and analgesic effect assessment in an alteration which also affects human: gout arthritis, and because of the advantages that this model presents over other experimental models used in preclinics. Used doses in PIFIR model to obtain CT from Betamethasone and Methocarbamol were selected after performing some pharmacological research tests to located effective dosage ranges, in rat (PIFIR model). Doses also appear after performing log increases from 0.25 or 0.50 log units.

Data obtained confirm that Betamethasone administered in combination with Methocarbamol possess a very useful anti-inflammatory and analgesic activity in conditions of gout arthritis, in this case in the rat.

### CONCLUSIONS

As to CT efficacy: the combination of Betamethasone + Methocarbamol showed higher efficacy than that shown by the same compounds but when individually administered.

As to action start, it was significantly improved with the administration of the combined active principles.

As to anti-inflammatory and analgesic effect duration, effects were analyzed continuously along 5 hours and these were kept quite satisfactorily even after time elapsed.

As to Emax (maximum effect), the combination of Betamethasone + Methocarbamol showed in CT the highest Emax levels.

As to global anti-inflammatory and analgesic efficacy (AUC) assessed along 5 continuous hours, the combination of Betamethasone + Methocarbamol showed better effects than the individual administration of each compound.

Under the inflammation and pain experimental conditions established in the experimental model, the combination of Betamethasone + Methocarbamol produced a significant improvement in anti-inflammatory and analgesic efficacy upon comparing with the individual delivery of each compound (p<0.05).

## Claims

1. A pharmaceutical composition **characterized in that** comprises a synergistic combination of a steroidal anti-inflammatory agent, such as active principle: Betamethasone,' which is present in the formulation in form of its phosphate and dipropionate pharmaceutically acceptable salts, and a centrally acting muscle relaxant, such as active principle: Methocarbamol, in addition to pharmaceutically acceptable excipients; wherein active principles are present in the formulation in a concentration range from 2.0 mg to 4.0 mg for Betamethasone phosphate, from 5.0 mg to 10.0 mg for Betamethasone dipropionate and from 100.0 mg to 750.0 mg for Methocarbamol, which are formulated in a single dosage unit to be intramuscularly administered, indicated for control and treatment of musculoskeletal inflammatory diseases associated with muscle contracture.

2. A pharmaceutical composition according to claim 1, **characterized in that** the steroidal anti-inflammatory agent, such as active principle: Betamethasone, in form of its phosphate pharmaceutically acceptable salt, is present in the formulation in a concentration range from 2.0 mg to 4.0 mg, being preferably used a concentration formulation of 2.0 mg per dose unit.

3. A pharmaceutical composition according to claims 1 and 2, **characterized in that** steroidal anti-inflammatory agent, such as active principle: Betamethasone, in form of pharmaceutically acceptable dipropionate salt is present in the formulation in a concentration range from 5.0 mg to 10.0 mg, being preferably used a concentration formulation of 5.0 mg per dose unit.

4. A pharmaceutical composition according to claims 1 to 3, **characterized in that** the centrally acting muscle relaxant, such as active principle: Methocarbamol, is present in the formulation in a concentration range from 100.0 mg to 750.0 mg, being preferably used a concentration formulation of 400.0 mg per dose unit.

5. A pharmaceutical composition according to claims 1 to 4, **characterized in that** is formulated in a single dosage unit to be intramuscularly administered in the form of solution and suspension.

6. The use of the pharmaceutical composition according to claims 1 to 5, **characterized in that** is indicated for control and treatment of musculoskeletal inflammatory diseases associated with muscle contracture.

7. A pharmaceutical composition according to claims 1 to 6, **characterized in that** demonstrates an important potency and efficacy to prevent or to decrease the symptomatology which is present together with musculoskeletal inflammatory diseases, such as: intense pain, inflammatory process and muscle contracture progression, in addition to time reduction wherein a larger therapeutic effect is manifested and prevention of appearance of side effects such as drowsiness and ambulatory impairment to perform daily activities.
